Europäisches Patentamt

European Patent Office

Office européen des brevets

(19)

(11) Publication number: **0 023 148**
**B1**

(12) **EUROPEAN PATENT SPECIFICATION**

(45) Date of publication of patent specification: **15.08.84**

(21) Application number: **80302463.7**

(22) Date of filing: **21.07.80**

(51) Int. Cl.³: **A 47 C 27/08,**
**A 47 C 31/12**

(54) Mattresses.

(30) Priority: **23.07.79 GB 7925575**

(43) Date of publication of application:
**28.01.81 Bulletin 81/4**

(45) Publication of the grant of the patent:
**15.08.84 Bulletin 84/33**

(84) Designated Contracting States:
**AT BE CH DE FR IT LI NL SE**

(56) References cited:
**US - A - 2 542 781**
**US - A - 2 604 641**
**US - A - 4 092 750**

(73) Proprietor: **Paterson, John Kirkpatrick**
**"Mariners" Alwalton**
**Nr. Peterborough Cambs (GB)**

(72) Inventor: **Paterson, John Kirkpatrick**
**"Mariners" Alwalton**
**Nr. Peterborough Cambs (GB)**

(74) Representative: **Colgan, Stephen James et al,**
**CARPMAELS & RANSFORD 43 Bloomsbury**
**Square**
**London WC1A 2RA. (GB)**

Courier Press, Leamington Spa, England.

## Description

This invention relates to mattresses, and particularly to those where the support for a body is provided by a low-pressure fluid rather than by springs.

It has long been accepted that spinal posture in sleep is important in relation to the incidence of pain over the whole length of the spine. The sagging springs of the Victorian era, coupled with over-soft mattresses, produced a hammock-like effect, thoroughly bad for the human spine. Largely as a reaction to this, the so-called orthopaedic bed was evolved, seeking in the main to provide a more or less flat base with a relatively firm mattress, the antithesis of the sagging bed of the past.

While this is an improvement on the Victorian bed, it does nor cater for changes in posture during sleep, this being something almost universally obtaining in man, usually very frequent. Lying supine, the "orthopaedic" bed provides a firm platform, satisfactory from a postural point of view, though not providing uniform support for the body. Lying on either side, the "orthopaedic" bed, by the very nature of its shape and firmness, induces a highly undesirable posture of multiple scoliosis, which if maintained for long periods, may provoke spinal derangement.

It is thus clear that the currently accepted concept of the orthopaedic bed is fundamentally unsound, and that what is required is a bed which will automatically change in contour with alterations in posture, while maintaining uniform support for the body. On lying supine, the ideal bed must remain firm and relatively flat, while on turning to either side, a satisfactory posture may only be maintained by a relatively mountainous contour in the bed. It is clear that such a facility for radical change of contour is met in the water bed. The chief disadvantages of this are — its initial cost, its weight, its maintenance, and the excessive, undesirable rapidity of change of shape.

The last of these disadvantages could be met by using a fluid very much more viscous than water. This, however, would remain unsatisfactory on account of the other disadvantages.

The present invention provides mattress for orthopaedic use and which employs a plurality of cores of a cellular structure inflated with a gas such as air. The cores are encased in a mattress cover and can be inflated to different pressures if desired. In the past there have been numerous suggestions for various forms of airbeds. For example, US—A—4092720 decribes an airbed in which cores are of a cellular structure, but the cells extend the length of the cores. Movement of air along the length of the core is rapid and the firm support necessary in an orthopaedic bed is not provided. The airbed of US—A—4092720 is, in fact, concerned with improved insulating properties for use in exposed conditions.

US—A—2542781 also describes an airbed or inflatable mattress, this consists of a single cellular core in an outer cover. Again, it is not specifically intended for orthopaedic use and the possibility of using a plurality of cores which may be inflated to varying pressures is not described.

In accordance with the invention there is provided a mattress having:

(1) a plurality of fluid-tight elongate cores each extending parallel to one another along the length of the mattress, each core enclosing a cellular structure comprising a plurality of cells extending end to end along the core, the volume of each cell being from 120 to 20,000 cm³ and adjacent cells in each core communicating with each other by means of perforations in the cell walls, which perforations permit a limited rate of flow of fluid between the cells in each core, the cores not being in fluid communication with one another; and

(2) a mattress cover fitting around said cores and having partitions forming channels into which the cores fit.

The volume of any cell is much larger than the cell volume obtained in foamed plastics materials (often used in cheaper bedding for caravans, boats, etc) but less than the cell volume obtained in the commonly-available air-beds, where one cell typically forms a pillow area and normally about four parallel, longitudinally-extending cells inter-communicating will be a gas at low pressure, typically air at just bed. The individual cell volumes in the mattresses in accordance with this invention will be from 120 cm³ to 20,000 cm³.

Preferably the fluid employed in the mattress will bea gas at low pressure, typically air at just 10—50 KPa above atmospheric pressure. Preferably means are provided for inflating the mattress — e.g. through a bicycle wheel-type air valve.

The mattress comprises a number of said cores which fit into a mattress cover having partitions to provide channels for the cores. The cores are generally elongate in shape and designed to fit into the mattress cover so that the cores extend parallel to one another and from the head to the foot of the mattress.

A preferred embodiment of the invention will now be described.

The mattress consists of a plurality of airtight cores each approximately 200 cms × 22 cms × 15 cms. Each core is mainly composed of a cellular structure, each cell being approximately 14 cms × 4 cms × 4 cms. The cell walls include perforations to permit slow movement of air between the cells. The core is provided with a bicycle-wheel-type air valve which opens into an ante-chamber itself communicating with the cells of the core. The core may be made of any appropriate pliable gas-tight material, for instance a plastics-coated fabric.

Individual cores are fitted into a mattress cover itself provided with longitudinal partitions providing channels to accommodate one core each. The top and bottom of the cover are padded in the usual way. Six cores are required for a standard-size double mattress and four cores for a single mattress.

This arrangement meets the requirements of the human spine in repose. It provides uniform support for the body, regardless of posture. It changes shape in order to accommodate changes in posture, automatically, but slowly, thus not producing undesirable "bounce".

Its production cost may remain reasonably low. Its maintenance cost is kept relatively low, as the pressures required are very small, and individual cores may be replaced or repaired, rather than the whole mattress. Its weight is low. A further advantage is the ease of adjustment of the mattress to suit persons of widely differing weights; this even being applied to the two sides of a double mattress, so producing a "his and hers" effect. Alterations in firmness of the mattress may be made in the home, if desired, by use of a bicycle pump.

## Claims

1. A mattress having:

(1) a plurality of fluid-tight elongate cores each extending parallel to one another along the length of the mattress, each core enclosing a cellular structure comprising a plurality of cells extending end to end along the core, the volume of each cell being from 120 to 20,000 cm³ and adjacent cells in each core communicating with each other by means of perforations in the cell walls, which perforations permit a limited rate of flow of fluid between the cells in each core, the cores not being in fluid communication with one another; and

(2) a mattress cover fitting around said cores and having partitions forming channels into which the cores fit.

2. A mattress according to claim 1 wherein each core includes a gas-valve to enable it to be inflated with gas.

3. A mattress according to claim 2 wherein each core is provided with a said gas-valve which communicates with an antechamber itself communicating with the said cells.

## Revendications

1. Matelas comportant:

(1) plusieurs longs noyaux étanches au fluide qui s'étendent chacun parallèlement les uns aux autres dans le sens de la longueur du matelas, chaque noyau renfermant une structure cellulaire comprenant plusieurs cellules qui s'étendent bout à bout dans le noyau, le volume de chaque cellule étant compris entre 120 et 20.000 cm³ et des cellules adjacentes de chaque noyau communiquant l'une avec l'autre par des perforations prévues dans les parois des cellules, ces perforations permettant un débit d'écoulement de fluide limité entre les cellules dans chaque noyau, les noyaux n'étant pas en communication de fluide l'un avec l'autre; et

(2) une enveloppe de matelas qui s'ajuste autour des noyaux et qui comporte des cloisons formant des chenaux dans lesquels les noyaux s'ajustent.

2. Matelas suivant la revendication 1, caractérisé en ce que chaque noyau comprend une valve permettant de la gonfler au moyen de gaz.

3. Matelas suivant la revendication 2, caractérisé en ce que chaque noyau est pourvu d'une valve de gaz qui communique avec une antichambre communiquant elle-même avec les cellules.

## Patentansprüche

1. Matratze mit: (1) einer Mehrzahl fluiddichter länglicher Kerne, die sich jeweils parallel zueinander entlang der Matratzenlänge erstrecken und je eine aus einer Mehrzahl sich von einem zum anderen Ende entlang des Kerns erstreckender Zellen bestehende Zellenstruktur einschleissen, wobei das Volumen der Zellen jeweils 120 bis 20 000 cm³ beträgt und nebeneinanderliegende Zellen in jedem Kern miteinander über Löcher in den Zellenwänden in Verbindung stehen, wobei die Löcher einen beschränkten Fluiddurchfluss zwischen den Zellen in jedem Kern zulassen und die Kerne nicht miteinander in Fluidverbindung stehen, und (2) einer diese Kerne umgebenden Matratzenhülle mit Trennwänden, die Kanäle bilden, in welche die Kerne hineinpassen.

2. Matratze nach Anspruch 1, worin jeder Kern ein Gasventil besitzt, damit er mit Gas aufgeblasen werden kann.

3. Matratze nach Anspruch 2, worin jeder kern mit einem solchen Gasventil versehen ist, das mit einer Vorkammer verbunden ist, die ihrerseits mit diesen Zellen in Verbindung steht.